# EUROPEAN PATENT SPECIFICATION

(11) **EP 1 582 186 B1**
(45) Date of publication and mention of the grant of the patent: **19.03.2008**
(21) Application number: 03702283.7
(22) Date of filing: 09.01.2003
(51) Int. Cl.: A61F 6/04, A61F 6/06, A61K 35/74

(54) **CONDOM COATED WITH LACTOBACILLUS COMPOSITIONS**
MIT LACTOBACILLUSZUSAMMENSETZUNGEN ÜBERZOGENES KONDOM
CONDOM REVETU DE COMPOSITIONS DE LACTOBACILLE

(43) Date of publication of application: 05.10.2005
(73) Proprietor: Bioleader Technology Corp., Xinzhuang City Taipei County 242 (TW)
(72) Inventor: WANG, Chih-Hong, 10F., No. 171, Siyuan Rd.,, Taipei Country 242, Taiwan (CN); CHEN, Hsin-Chung, 10F., No. 171, Siyuan Rd.,, Taipei Country 242, Taiwan (CN); CHENG, Yu-Shen, 10F., No. 171, Siyuan Rd.,, Taipei Country 242, Taiwan (CN)
(74) Representative: Viering, Jentschura & Partner
(86) International application number: PCT/CN2003/000016
(87) International publication number: WO 2004/062538

(56) References cited:
- EP-A- 0 661 028
- WO-A-01/95951
- AU-B2- 679 310
- CN-A- 1 200 934
- CN-A- 1 348 746
- JP-A- 11 192 252
- US-A- 5 466 463
- US-A- 5 778 886
- US-A- 5 804 179
- US-A- 5 878 747
- US-A- 6 074 671

## Description

### BACKGROUND OF THE INVENTION

### 1. Field of the Invention

The present invention relates to a condom coated with lactobacillus compositions comprising natural or genic recombined lactobacillus for constructing, maintaining and reconstructing non-pathogenic, natural or genic recombined colonies in vagina to prevent vaginal pathogenic microbial infection and viral infection.

### 2. Description of Prior Art

Microbicidal and spermicidal compositions have been widely used in the prior art in creams, foams, suppositories by themselves and also in conjunction with various mechanical contraceptive devices, primarily for the purpose of contraception. Nonoxynol 9, a spermicidal agent, has also been reported to have certain bactericidal action and capable of killing the human immunodeficiency virus (HIV) in the prior art. However, the use of spermicidal and bactericidal agent, such as nonoxynol 9, in condom or other vaginally inserted suppositories is not without problems inasmuch as there agents tend to destroy or diminish the healthy bacterial flora of the vagina, and cause for the woman a risk to develop other microbial infections, such as yeast infection.

The condom is the most effective and most frequently using contraceptive method. Using the condom also helps prevent sexually transmitting disease by interposing between the female and male, most importantly between the uterine cervix and the glands and orifice of the penis. However, the spermicidal agents and the latex material of the condoms can erode the mucosa if used too frequently, and even with infrequent use, can disrupt the protection of vaginal healthy bacterial flora. Although the prior art has recognized the need to maintain or reestablish a healthy intra-vaginal bacterial flora, the only solution provide to this problem in the prior art was in the form of douches or other types of vaginal cleaning systems which contained a colony of *Lactobacillus acidophilus* (see, for example, patent US 5466463)*.* These douches or other vaginal cleaning systems are inconvenient, and they also contribute nothing to contraception, nor do they protect against transmission of sexually transmitted diseases during heterosexually intercourse.

Lactobacillus such as *Lactobacillus casei* and *Lactobacillus fermentum* have been reported to have healthy action in vagina is not only in the natural form but also can be genetically modified. The prior art has indicated that a lactobacillus can be genetically modified to express the polypeptide or carbohydrate moiety on the bacterial surface or in the intracellular environment, which have the ability to bind, inactive or remove the pathogenic microbial from vagina and also have the ability to act on the immunity reaction of human to against the viral infection in vagina, such as human papillomavirus (HPV).

### SUMMARY OF THE INVENTION

It is an object of the present invention to provide a condom coated with lactobacillus compositions for enhancing the effect of contraception and preventing venereal infection, and for constructing, maintaining and reconstructing non-pathogenic, natural or genic recombined colonies in vagina to prevent vaginal pathogenic microbial infection and viral infection.

It is another object of the present invention to provide a method for the manufacture of a condom coated with lactobacillus compositions.

The compositions coated on the condom of this present invention comprise lactobacillus made in the form of powder or other forms, such as emulsion, foam, micro-encapsulated form, and comprise at least one kind of living lactobacillus strain, and selectively comprise one kind of lubricant.

When the micro-encapsulated form has been used, the living lactobacillus strains are micro-encapsulated, which can maintain the viability of the lactobacillus and also protect the living lactobacillus strain from the action of the antimicrobial or the other compositions during the "shelf-life" of the condom presented in the invention. The coating or substance which encapsulates the bacteria on the other hand is of such material which releases the bacteria in the vaginal milieu primarily due to the effect of moisture. The released lactobacillus serve to maintain or reestablish the healthy flora on the vaginal mucosa, and excrete hydrogen peroxide and other bactericidins which suppress pathogenic microbial that promote infections.

The lactobacillus strain may be natural or genic recombined lactobacillus including acidophilic bacteria, bifidobacteria, the species *Lactococcus lactis, Lactobacillus rhamnosus, Lactobacillus fermentum, Lactobacillus brevis, Lactobacillus plantarum, Lactobacillus casei, Lactobacillus delbruckii* sbp. *bulgaricus, Lactobacillus delbruckii* sbp*. lactis, Streptococcus thermophilus, Streptococcus gordonii or Leuconostoc mesenteroides.*

### BRIEF DESCRIPTION OF THE DRAWINGS

The drawings disclose an illustrative embodiment of the present invention which serves to exemplify the various advantages and objects hereof, and are as follows:
FIG. 1 is a diagrammatic representation of a condom, partially cut-away to show a condom coated with lactobacillus compositions on outside;
FIG. 2 is a cross-sectional drawing to a portion of the condom shown in FIG. 1;
FIG. 3 is a diagrammatic representation, partially cut-away to a rolled condom shown in FIG. 1; and
FIG. 4 is a diagrammatic representation of a condom, partially cut-away to shown a condom coated with lactobacillus compositions on inside and outside.

### DETAILED DESCRIPTION OF THIS INVENTION

The present invention is a condom providing contraceptive effect, sexually transmitted disease prevention and vaginal healthy improvement, which is coated with lactobacillus compositions containing living lactobacillus strain.

The lactobacillus compositions comprise lactobacillus strains are prepared in the form of powder, or other forms, such as emulsion, foam, micro-encapsulated form to protect them from the action of other antimicrobial agent. Additionally, all the lactobacillus strain used in the present invention may be purchased from commercial sources, or may be obtained from laboratory strains, and the lactobacillus strain using in this present invention may be natural or genic recombined lactobacillus.

The living lactobacillus strain is the essential component of the condom of this present invention, which shall be acidophilic bacteria, bifidobacteria, the species *Lactococcus lactis, Lactobacillus rhamnosus, Lactobacillus fermentum, Lactobacillus brevis, Lactobacillus plantarum, Lactobacillus casei, Lactobacillus delbruckii* sbp*. bulgaricus, Lactobacillus delbruckii* sbp. *lactis, Streptococcus thermophilus, Streptococcus gordonii* or *Leuconostoc mesenteroides,* and should be present at least in the range of 10³ living lactobacillus strain per condom, a more preferred range being 10⁵ to 10⁷, and the preferred number of living lactobacillus strain being approximately 1 million (10⁶) per condom.

As is know in the prior art, all the lactobacillus mentioned above are "friendly" bacteria, and form the healthy flora in the human body. Some of these bacteria are known to produce certain bactericidins and hydrogen peroxide, which helps to suppress pathogenic bacteria, called the probiotic lactobacillus. The probiotic lactobacillus are, to this effect, of particular interest within the framework of the present invention. These bacteria are in fact capable of adhering to human vaginal mucosa, of excluding pathogenic bacteria on human vaginal mucosa, and/or of acting on the human immune system by allowing it to react more strongly to external aggression (immunomodulatory capacity).

Additionally, the lactobacillus used on the lactobacillus compositions of the invention may be freeze-dried and micro-encapsulated in the separation substance comprising solidified fatty acid, sodium alginate, polymerized hydroxypropylmethylcellulose, polymerized polyvinylpyrrolidone, or mixtures thereof, in order to protect them from the action of other antimicrobial agent, such as nonoxynol 9.

In addition to the lactobacillus compositions mentioned above, the condom of the invention may be further coated with other compositions including non-essential ingredients or components, such as spermicidal and bactericidal agent or agents. These components are considered non-essential because the basic objectives of the invention can be attained without them. Nevertheless, the embodiments of the invention which include these non-essential components offer certain advantages, and are therefore considered the preferred embodiments, and should also be considered novel and innovation, in their combination, to the basic embodiments.

A method of coating lactobacillus compositions on a condom, if necessary, comprises coating the whole surface or necessary portion of a condom by dropping, dipping, coating or spraying a solution containing a lubricant as described before. In this way, a condom having the effect for constructing, maintaining and reconstructing non-pathogenic, natural or genic recombined colonies in vagina and additionally having a lubricating effect shall be obtained.

On a condom manufactured in a usual method, for example, a condom which is stripped off from a condom-former and rolled up after the forming process followed by examination process, a composition containing lactobacillus is added and the condom is packaged with film to be spread with lactobacillus compositions on the whole surface thereof. As a healthy component, a powder form lactobacillus compositions added with a lubricant as described before may be used. In this way, a wet-type of product coated with a multi-beneficial effect in a creamy state shall be obtained.

In another method, a condom can be taken from a water vessel (pinhole examination vessel) remaining on the condom former as described before, dipped in a dipping vessel (liquid vat) containing a solution of the composition which comprises lactobacillus having multi-beneficial effect to be coated with lactobacillus compositions on the whole surface thereof, taken from the dipping vessel, dried, stripped off from the former and rolled up.

A solution of lactobacillus compositions to a dipping vessel can be a gelled solution of lactobacillus compositions together with a lubricant, for example, isopropyl alcohol, as described before. In this way, a dried product coated with a multi-beneficial agent can be obtained.

In addition, on a condom, produced like this silicone oil or an aqueous solution of a lubricant can be added, followed by film packaging thereof.

The present invention is further explained by the following examples, but these examples are illustrated to exemplify the present invention and the scope of the present invention is not restricted by these examples.

### EXAMPLES

### Lactobacillus Preparation

At first, a concrete example of preparation of lactobacillus compositions used in the present invention is exemplified. The lactobacillus of the present invention can be grown in any medium which provides effective growth of the microbe without contamination, loss of genetic stability, or loss of any other desirable characteristics of the lactobacillus strains. More particularly, the lactobacillus strains of the present invention are grown in a culture medium which includes a source of assimilable organic carbon, a source of assimilable nitrogen and appropriate salts and trace metals. A preferred medium for culturing *Lactobacillus* strains of the present invention is MRS medium.

The lactobacillus microorganisms of the present invention can be cultured in conventional culture conditions, which include, but are not limited to agar surface culture or broth fermentation. Both agar surface culture and broth fermentation methods are well known in the art. The *Lactobacillus* are preferably cultured anaerobically or microaerophilically.

The temperature of the culture medium can be any temperature suitable for growth of the lactobacillus strains. For example, prior to inoculation of the culture medium with an inoculum, the culture medium can be brought to and maintained at a temperature in the range of about 20° C.-35° C., and more preferably about 25° C.-35° C.

The culture medium is inoculated with an actively growing culture of the lactobacillus strains of the present invention in an amount sufficient to produce, after a reasonable growth period, a suitable cell density for transfer to the next drying process, such as freeze drying or spay drying, which is in order to remove the medium. Typical inoculation cell densities are within the range of about 10⁶-10⁹ CFUs/ml, and more preferably about 10⁸-10⁹ CFUs/ml, based on the dry weight of the cells. The cells are then grown to a cell density in the range of about 10⁷-10⁹ CFUs/ml, and more preferably to about 10⁸ CFUs/ml. At this stage, the cells are harvested for the next drying and lyophilized process.

### Encapsulation Method

Living, lyophilized lactobacillus that have been lyophilized after the removal of the media are used for encapsulation. The bacteria can be obtained from commercial sources, or can be obtained from laboratory strains. The organisms are grown to log phase in nutrient media. Suitable media include Thayer-Martin media, Trypticase Soy, Brain-Heart Infusion Broth, or any other enriched media suitable for the cultivation of these organisms, as no particular media is critical to the success of this suppository. The only important factors are the viability and quantity of the micro-organisms that are always determined by standard clinical laboratory dilution methods, such as plating the quantified dilution of bacteria on to blood agar plates or other enriched media, incubating at 37° C. for 24-48 hours in a 5-10% carbon dioxide atmosphere, and then performing a colony count. The removal of the nutrient media is done by centrifugation at 14,000.times.g at 0-4° C., and then washing with sterile, balanced salts and 5% glucose solution at least three times after the initial centrifugation. The bacteria are then "snap frozen" with liquid nitrogen and then lyophilized under high vacuum.

The freshly obtained, washed and lyophilized bacteria obtained as described above are suspended in 10 ml of 5% glucose saline solution in such volume so as to obtain a heavy suspension of bacteria which contains between one to ten billion organisms per ml, at 0-4° C. All of these procedures are performed in the 0-4° C. temperature range unless otherwise noted, in order to maintain viability of the lactobacilli bacteria which at room temperature lose viability. The suspension of bacteria is rapidly, but gently, stirred while 0.2-0.4 ml of sodium alginate solution (1.5% weight by volume) is added. The above mixture is then transferred into a 4 liter round bottom flask by using a nitrogen stream through a sheathed 14 gauge needle. The 4 liter round bottom flask was previously washed with a 5% albumin solution, and thereafter heated for at least 10 hours at 65° C., and the needle and the tubing used in the process have also been treated this way.

Thereafter the above mixture is forced through a 30 gauge multi-beveled needle under pressure using a large syringe and nitrogen stream. Very small droplets are generated at the end of the needle which are dried by the nitrogen and air stream around the 30 gauge needle, and the droplets are collected in an aqueous solution of 1.3-2% calcium chloride where they gel. Thereafter, they are washed at least three times with 0.08-0.13% 2-(N-cyclohexyl-amino) ethanesulfonic acid (CHES) solution and 1.0-1.5% calcium chloride solution.

The gelled droplets or little spheres are further washed with at least a five fold excess of the 0.1% CHES 1.1% calcium chloride, and normal saline solution. The resultant spheres are then "snap frozen" in liquid nitrogen and then lyophilized. After these steps, the encapsulated organisms can be used in the formulations of the present invention.

As an improvement over encapsulation method mentioned above, the following further steps are performed to render the bacteria more resistant to the cationic antimicrobials. The steps are performed at 0-4° C. Thus, after the washings described in encapsulation method mentioned above, the materials are reacted with poly L-lysine (Sigma) solution (0.05% w/v) spheres for ten minutes. The spheres are then washed with normal saline buffered to pH 4.5 with lactic acid. The resultant spheres are then "snap frozen" in liquid nitrogen and then lyophilized. After these steps, the encapsulated organisms can be used in the formulations of the present invention.

### Condom Coating

### Example 1

The lactobacillus compositions was coated on a condom which was formed, examined, stripped off and rolled up, followed by heat-sealing thereof. Then these compositions gradually permeated and spread over the whole surface of the condom and a coated condom with wet and suitable lubricating properties was obtained.

### Example 2

An aqueous solution of .lambda.-carrageenan mixed with isopropyl alcohol was put in a liquid vat and a condom (while still covering a condom-former) was dipped therein after the examination for pinholes.

The condom was taken from the liquid vat, dried, stripped off from the former and rolled up. In this way a condom coated homogenously with the lactobacillus contained compositions was obtained.

### Example 3

An aqueous solution of a lubricant consisting of propylene glycol, sodium alginate and glycerine was coated on the condom obtained in example 2, followed by heat-sealing to make the condom coated homogeneously with the lactobacillus contained compositions and further with a lubricant.

The drawings disclose an illustrative embodiment of the present invention.

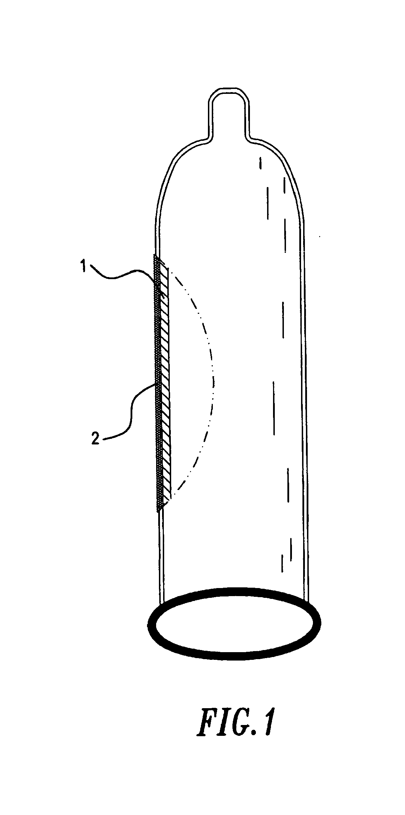

FIG. 1 is an illustration, in partial cross-section, of a condom 1 coated on the outside with a layer 2 comprising a mixture of lubricant and the lactobacillus compositions.

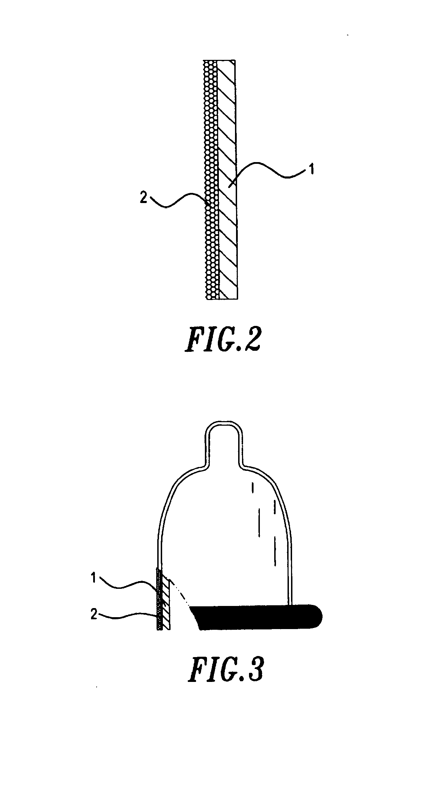

FIG. 2 is a cross-section of a portion of a condom 1 coated with a layer 2 comprising the lactobacillus compositions. The coating is thinner than the condom 1.

FIG. 3 is an illustration, in partial cross-section, of a rolled condom 1 coated on the outside with a layer comprising a mixture of lubricant and the lactobacillus compositions.

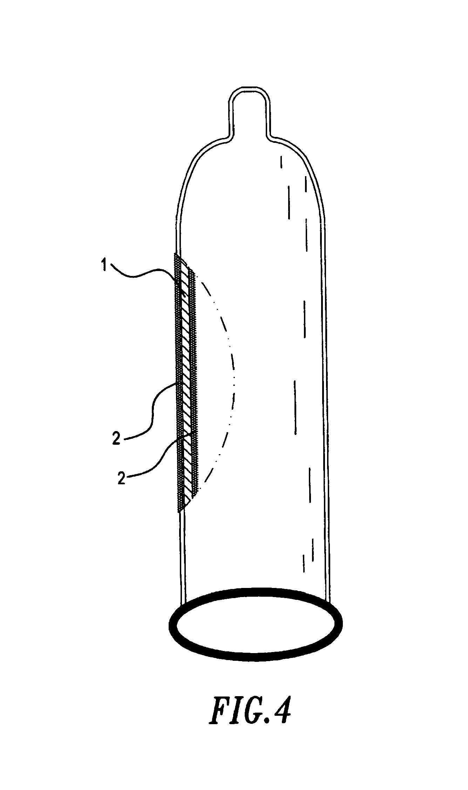

FIG. 4 is an illustration in partial cross-section of a condom 1 coated with a layer 2 comprising the lactobacillus compositions, on the inside and the outside of the condom.

Many changes and modifications in the above described embodiment of the invention can, of course, be carried out without departing from the scope thereof. Accordingly, to promote the progress in science and the useful arts, the invention is disclosed and is intended to be limited only by the scope of the appended claims.

## Claims

1. A condom coated with a layer (2) comprising lactobacillus compositions on outside or on inside and outside of the condom (1) for constructing, maintaining and reconstructing non-pathogenic, natural or genic recombined colonies in vagina, **characterized in that** the layer (2) comprising at least in the range of 10³ living lactobacillus strain.

2. The condom of claim 1, **characterized in that** the condom (1) is male condom or female condom

3. The condom of claim 1, **characterized in that** the lactobacillus is natural lactobacillus strain.

4. The condom of claim 1, **characterized in that** the lactobacillus is genic recombined lactobacillus.

5. The condom of claim 1, **characterized in that** the lactobacillus strain is selected from a group consisting of acidophilic bacteria, bifidobacteria, Lactococcus lactis, Lactobacillus rhamnosus, Lactobacillus fermentum, Lactobacillus brevis, Lactobacillus plantarum, Lactobacillus casei, Lactobacillus delbruckii sbp. bulgaricus, Lactobacillus delbruckii sbp. lactis, Streptococcus thermophilus, Streptococcus gordonii, Leuconostoc mesenteroides and mixtures thereof.

6. The condom of claim 1, **characterized in that** the lactobacillus compositions comprise at least a kind of lactobacillus strain.

7. The condom of claim 1, **characterized in that** the lactobacillus strain adheres to human vaginal cells to prevent vaginal pathogenic microbial infection and viral infection.

8. The condom of claim 1, **characterized in that** the lactobacillus compositions are made as a solution.

9. The condom of claim 1, **characterized in that** the lactobacillus compositions are made as a solid.

10. The condom of claim 1, **characterized in that** the lactobacillus compositions are made as a lubricant.

11. The condom of claim 1, **characterized in that** the lactobacillus compositions comprising lactobacillus made in the form of powder.

12. The condom of claim 1, **characterized in that** the lactobacillus compositions comprising encapsulated lactobacillus.

13. The condom of claim 12, **characterized in that** the encapsulated lactobacillus are micro-encapsulated in a separation substance selected from a group consisting of solidified fatty acid, sodium alginate, polymerized hydroxypropylmethylcellulose, polymerized polyvinylpyrrolidone, and mixtures thereof.

## Patentansprüche

1. Ein mit einer Schicht (2) beschichtetes Kondom, das Lactobacillus-Zusammensetzungen an der Außenseite oder an der Innenseite und Außenseite des Kondoms (1) zum Aufbauen, Erhalten und Wiederaufbauen nicht-pathogener, natürlicher oder genetisch rekombinierter Kolonien in der Vagina umfasst, **dadurch gekennzeichnet, dass** die Schicht (2) mindestens einen lebenden Lactobacillus-Stamm im Bereich von 10³ umfasst.

2. Das Kondom nach Anspruch 1, **dadurch gekennzeichnet, dass** das Kondom (1) ein männliches Kondom oder ein weibliches Kondom ist.

3. Das Kondom nach Anspruch 1, **dadurch gekennzeichnet, dass** der Lactobacillus ein natürlicher Lactobacillus-Stamm ist.

4. Das Kondom nach Anspruch 1, **dadurch gekennzeichnet, dass** der Lactobacillus genetisch rekombinierter Lactobacillus ist.

5. Das Kondom nach Anspruch 1, **dadurch gekennzeichnet, dass** der Lactobacillus-Stamm aus einer Gruppe, die aus acidophilen Bakterien, Bifidobakterien, *Lactococcus lactis, Lactobacillus rhamnosus, Lactobacillus fermentum, Lactobacillus brevis, Lactobacillus plantarum, Lactobacillus casei, Lactobacillus delbruckii sbp. bulgaricus, Lactobacillus delbruckii sbp. lactis, Streptococcus thermophilus, Streptococcus gordonii, Leuconostoc mesenteroides* und Mischungen davon besteht, ausgewählt wird.

6. Das Kondom nach Anspruch 1, **dadurch gekennzeichnet, dass** die Lactobacillus-Zusammensetzungen mindestens eine Art eines Lactobacillus-Stamms umfassen.

7. Das Kondom nach Anspruch 1, **dadurch gekennzeichnet, dass** der Lactobacillus-Stamm an menschliche Vaginalzellen anhaftet, um eine vaginale pathogene mikrobielle Infektion und eine Virusinfektion zu verhindern.

8. Das Kondom nach Anspruch 1, **dadurch gekennzeichnet, dass** die Lactobacillus-Zusammensetzungen als eine Lösung hergestellt werden.

9. Das Kondom nach Anspruch 1, **dadurch gekennzeichnet, dass** die Lactobacillus-Zusammensetzungen als ein Feststoff hergestellt werden.

10. Das Kondom nach Anspruch 1, **dadurch gekennzeichnet, dass** die Lactobacillus-Zusammensetzungen als ein Gleitmittel hergestellt werden.

11. Das Kondom nach Anspruch 1, **dadurch gekennzeichnet, dass** die Lactobacillus-Zusammensetzungen Lactobacillus umfassen, der in Form eines Pulvers hergestellt wird.

12. Das Kondom nach Anspruch 1, **dadurch gekennzeichnet, dass** die Lactobacillus-Zusammensetzungen verkapselten Lactobacillus umfassen.

13. Das Kondom nach Anspruch 12, **dadurch gekennzeichnet, dass** die verkapselten Lactobacillus in einer Trennsubstanz mikroverkapselt werden, die aus einer Gruppe, die aus verfestigter Fettsäure, Natriumalginat, polymerisierter Hydroxypropylmethylcellulose, polymerisiertem Polyvinylpyrrolidon und Mischungen davon besteht, ausgewählt wird.

## Revendications

1. Préservatif recouvert d'une couche (2) comprenant des compositions de lactobacilles à l'extérieur ou à l'intérieur et à l'extérieur du préservatif (1) pour construire, maintenir et reconstruire des colonies non pathogènes, naturelles ou obtenues par recombinaison génique dans le vagin, **caractérisé en ce que** la couche (2) comprend au moins une couche de l'ordre de 10³ lactobacilles vivants.

2. Préservatif selon la revendication 1, **caractérisé en ce que** le préservatif (1) est un préservatif pour homme ou un préservatif pour femme.

3. Préservatif selon la revendication 1, **caractérisé en ce que** le lactobacille est une souche de lactobacille naturelle.

4. Préservatif selon la revendication 1, **caractérisé en ce que** le lactobacille est une souche de lactobacille obtenue par recombinaison génique.

5. Préservatif selon la revendication 1, **caractérisé en ce que** la souche de lactobacille est choisie dans le groupe constitué des bactéries acidophiles, des bifidobactéries, des Lactococcus lactis, Lactobacillus rhamnosus, Lactobacillus fermentum, Lactobacillus brevis, Lactobacillus plantarum, Lactobacillus casei, Lactobacillus delbruckii sous-espèce bulgaricus, Lactobacillus delbruckii sous-espèce lactis, Streptococcus thermophilus, Streptococcus gordonii, Leuconostoc mesenteroides et leurs mélanges.

6. Préservatif selon la revendication 1, **caractérisé en ce que** les compositions de lactobacilles comprennent au moins un type de souche de lactobacille.

7. Préservatif selon la revendication 1, **caractérisé en ce que** la souche de lactobacille adhère aux cellules vaginales humaines pour prévenir l'infection microbienne pathogène et l'infection virale du vagin.

8. Préservatif selon la revendication 1, **caractérisé en ce que** les compositions de lactobacilles sont préparées sous la forme d'une solution.

9. Préservatif selon la revendication 1, **caractérisé en ce que** les compositions de lactobacilles sont préparées sous la forme d'un solide.

10. Préservatif selon la revendication 1, **caractérisé en ce que** les compositions de lactobacilles sont préparées sous la forme d'un lubrifiant.

11. Préservatif selon la revendication 1, **caractérisé en ce que** les compositions de lactobacilles comprennent des lactobacilles préparés sous la forme d'une poudre.

12. Préservatif selon la revendication 1, **caractérisé en ce que** les compositions de lactobacilles comprennent des lactobacilles encapsulés.

13. Préservatif selon la revendication 12, **caractérisé en ce que** les lactobacilles encapsulés sont micro-encapsulés dans une substance de séparation choisie dans le groupe constitué de l'acide gras solidifié, de l'alginate de sodium, de l'hydroxypropylméthylcellulose polymérisée, de la polyvinylpyrrolidone polymérisée, et de leurs mélanges.
